# EUROPÄISCHE PATENTSCHRIFT

(11) **EP 4 267 019 B1**
(45) Veröffentlichungstag und Bekanntmachung des Hinweises auf die Patenterteilung: **02.04.2025**
(21) Anmeldenummer: 23707719.3
(22) Anmeldetag: 27.02.2023
(51) Int. Cl.: A61B 17/16, A61B 17/84

(54) **MEDIZINISCHE BOHRVORRICHTUNG UND MEDIZINISCHES BOHRSYSTEM**
MEDICAL DRILL DEVICE AND MEDICAL DRILL SYSTEM
DISPOSITIF MÉDICAL DE PERÇAGE ET SYSTÈME MÉDICAL DE PERÇAGE

(30) Priorität: 28.02.2022 DE 102022104674
(43) Veröffentlichungstag der Anmeldung: 01.11.2023
(73) Patentinhaber: Aesculap AG, 78532 Tuttlingen (DE)
(72) Erfinder: PEUKERT, Andreas, 78532 Tuttlingen (DE); BRUESSLER, Robert, 78532 Tuttlingen (DE); MARX, Irene, 78647 Trossingen (DE)
(74) Vertreter: Hoeger, Stellrecht & Partner Patentanwälte mbB
(86) Internationale Anmeldenummer: PCT/EP2023/054780
(87) Internationale Veröffentlichungsnummer: WO 2023/161460

(56) Entgegenhaltungen:
- US-A1- 2007 260 184
- US-A1- 2010 030 221
- US-A1- 2021 244 424

## Beschreibung

Die vorliegende Erfindung betrifft eine medizinische Bohrvorrichtung umfassend einen kanülierten Bohrer mit einem sich koaxial zur Längsachse des Bohrers erstreckenden Längskanal, wobei die Bohrvorrichtung an einem proximalen Ende einen Bohrvorrichtungkopplungsabschnitt umfasst zum drehfesten Koppeln mit einem Handgriff oder einer Bohrmaschine, wobei der Bohrer ein distales und ein proximales Ende aufweist, wobei die Bohrvorrichtung einen Mandrin umfasst mit einem distalen Mandrinabschnitt und einem proximalen Mandrinabschnitt, wobei der distale Mandrinabschnitt ausgebildet ist zum Einsetzen und Verschließen des Längskanals des Bohrers über die gesamte oder im Wesentlichen die gesamte Länge desselben in einer Bohrstellung und wobei der Bohrer im Bereich seines proximalen Endes einen Bohrerkopplungsabschnitt umfasst.

Ferner betrifft die vorliegende Erfindung ein medizinisches Bohrsystem umfassend mindestens eine medizinische Bohrvorrichtung.

In der Wirbelsäulenchirurgie werden heutzutage zahlreiche Eingriffe minimalinvasiv durchgeführt. Als Teil von Wirbelsäulenstabilisierungssystemen dienen insbesondere Pedikelschrauben, welche in die kortikale Schicht eines Wirbelbereichs des Pedikelkanals gesetzt werden. Es ist bekannt, die Kortikalis mit einem Bohrer zu eröffnen. In die vorgebohrte Kortikalis wird nach Entfernen des Bohrers ein Kirschner-Draht oder Bohr-Draht, nachfolgend als K-Draht bezeichnet, gesetzt, über den dann die Pedikelschraube an den Wirbel herangeführt werden kann, um diese in gewünschter Weise im Wirbel zu verankern. Ein Problem bei dieser Vorgehensweise ist, dass nach dem Entfernen des Bohrers der Eintrittspunkt in der Kortikalis mit dem K-Draht getroffen wird, denn es ist für den K-Draht hierfür keine Führung vorhanden. Zudem ist der minimal-invasive Zugang praktisch nicht einsehbar. Auch eine medizinische Bohrvorrichtung der eingangs beschriebenen Art mit einem kanülierten Bohrer hilft hierfür nichts, da beim Bohren Knochen in den Längskanal gedrückt wird, sodass der K-Draht nicht direkt durch den Bohrer platziert werden kann. Auch der kanülierte Bohrer muss daher zunächst entfernt werden, um dann in einem zweiten Schritt nach Entfernen des Bohrers den K-Draht zu setzen.

Die US 2010/0030221 A1 offenbart eine Klemmhülse zum Klemmen eines kanülierten Bohrers und einen Führungsdraht. Ein zurückziehbares Stilett und eine Kanülenanordnung sind aus der US 2007/0260184 A1 bekannt. Instrumentarien zum Befestigen an sowie zum Stabilisieren der Halswirbelsäule sind in der US 2021/0244424 A1 beschrieben.

Es ist daher eine Aufgabe der vorliegenden Erfindung, eine medizinische Bohrvorrichtung und ein medizinisches Bohrsystem der eingangs beschriebenen Art so zu verbessern, dass insbesondere deren Handhabung vereinfacht wird.

Diese Aufgabe wird bei einer medizinischen Bohrvorrichtung der eingangs beschriebenen Art erfindungsgemäß dadurch gelöst, dass der Bohrerkopplungsabschnitt zusammen mit dem proximalen Mandrinabschnitt den Bohrvorrichtungkopplungsabschnitt ausbildet.

Eine wie vorgeschlagen weitergebildete medizinische Bohrvorrichtung ermöglicht es insbesondere, bei einem minimal-invasiven Eingriff den Knochen zu eröffnen, wobei dann zum Setzen eines K-Drahts der Bohrer nicht entfernt werden muss. Vielmehr genügt es hier, den Mandrin aus dem Längskanal zu entfernen. Der Mandrin, welcher den Längskanal insbesondere formschlüssig vollständig oder nahezu vollständig ausfüllt, verhindert das Eindringen von Knochensubstanz in den Längskanal. Nach Entfernen des Mandrins ist dann der Längskanal des Bohrers vollständig frei von Knochensubstanz, sodass der Bohrer selbst als Führung für einen K-Draht genutzt werden kann, um diesen im Knochen, beispielsweise einem Wirbel, in definierter Weise zu platzieren, also in einer bestimmten Position und mit einer bestimmten Ausrichtung. Der Mandrin bildet also insbesondere ein Verschlusselement in Form eines Stopfens für den Längskanal, um zu verhindern, dass beim Bohren Knochensubstanz oder anderes Gewebe in den Längskanal eindringen und diesen verstopfen kann. Wie beschrieben muss also der Bohrer nicht wie bei herkömmlichen bekannten Verfahren vor dem Setzen des K-Drahts entfernt werden, sondern dient selbst als Führung für den K-Draht. So wird das Auffinden der Eintrittsstelle am Knochen beim Setzen des K-Drahts vereinfacht und damit auch die Handhabung der Bohrvorrichtung. Dies verbessert insbesondere die Qualität des chirurgischen Eingriffs. Ferner lässt sich so insbesondere auch eine Operationszeit verringern.

Günstig ist es, wenn der Bohrvorrichtungkopplungsabschnitt bezogen auf die Längsachse rotationsunsymmetrisch ausgebildet ist. So kann die Bohrvorrichtung in einer definierten Weise mit einem Handgriff oder einer Bohrmaschine, beispielsweise einem Bohrfutter beziehungsweise einer Kupplung derselben, gekoppelt werden. Insbesondere kann so eine drehfeste Kopplung zwischen dem Handgriff beziehungsweise der Bohrmaschine und der Bohrvorrichtung auf einfache Weise realisiert werden.

Vorteilhaft ist es, wenn der Bohrerkopplungsabschnitt mindestens eine Bohrermitnahmefläche aufweist, wenn der proximale Mandrinabschnitt mindestens eine Mandrinmitnahmefläche aufweist und wenn die mindestens eine Bohrermitnahmefläche und die mindestens eine Mandrinmitnahmefläche in der Bohrstellung aneinander anliegen oder im Wesentlichen aneinander anliegen. Insbesondere wenn die genannten Flächen parallel zur Längsachse verlaufen, ermöglichen sie es, den Bohrer um den Mandrin im Bereich des Bohrvorrichtungkopplungsabschnitt so miteinander in Eingriff zu bringen, dass beim Rotieren des Bohrers der Mandrin mitgedreht wird. Mit anderen Worten werden der Bohrer und der Mandrin auf diese Weise zusammen rotiert. Der proximale Mandrinabschnitt und der Bohrerkopplungsabschnitt bilden gegenseitig Mitnehmer, um Drehmomente auf die jeweils andere Komponente zu übertragen, und zwar insbesondere abhängig davon, auf welche Komponente vom Handgriff oder der Bohrmaschine Drehmomente eingeleitet werden.

Eine gute Kopplung und Führung von Mandrin und Bohrer lässt sich insbesondere dadurch erreichen, dass der Bohrerkopplungsabschnitt zwei Bohrermitnahmeflächen aufweist und dass der der proximale Mandrinabschnitt zwei Mandrinmitnahmeflächen aufweist. Insbesondere können die jeweils zwei Mitnahmeflächen jeweils paarweise parallel zueinander verlaufend ausgebildet sein. Beispielsweise können die zwei Mandrinmitnahmeflächen in entgegengesetzte Richtungen voneinander weg weisen, die zwei Bohrermitnahmeflächen können aufeinander zu weisen. Selbstverständlich ist es auch denkbar, eine solche Ausrichtung beziehungsweise Orientierung der Mitnahmeflächen umgekehrt zu realisieren.

Vorzugsweise weist der Längskanal einen ovalen Querschnitt auf. Insbesondere kann der Querschnitt kreisförmig sein. Ein ovaler Querschnitt hat zusätzlich den Vorteil, dass der Mandrin beim Rotieren des Bohrers zwangsweise mitrotiert wird. Ein kreisförmiger Querschnitt ist vorteilhaft, denn mit ihm lässt sich ein im Querschnitt kreisförmiger K-Draht in definierter Weise durch den Längskanal führen und auch in diesem verdrehen.

Günstig ist es, wenn der distale Mandrinabschnitt einen Querschnitt aufweist, welcher zum Querschnitt des Längskanals korrespondiert. So ist es insbesondere möglich, mit dem distalen Mandrinabschnitt den Längskanal formschlüssig auszufüllen. So kann das Eindringen von Knochensubstanz in den Längskanal auf einfache und sichere Weise verhindert werden.

Vorteilhafterweise ist am distalen Ende des distalen Mandrinabschnitts eine Spitze ausgebildet. Mit einer solchen Spitze lässt sich die Bohrvorrichtung einfach an einem Knochen platzieren. Insbesondere ein Abrutschen einer Spitze des Bohrers lässt sich so einfach und sicher vermeiden.

Gemäß einer weiteren bevorzugten Ausführungsform kann vorgesehen sein, dass ein distales Ende des distalen Mandrinabschnitts in der Bohrstellung bis an ein distales Ende des Mandrinkanals heranreicht oder über dieses vorsteht. In beiden Fällen kann insbesondere sichergestellt werden, dass der Längskanal distalseitig hinreichend verschlossen ist, um das Eindringen insbesondere von Knochengewebe beim bohrenden Eröffnen eines Knochens zu verhindern. Steht der distale Mandrinabschnitt über das distale Ende des Längskanals vor, kann er beispielsweise zum Positionieren des Bohrers am Knochen genutzt werden. Vorzugsweise ist das distale Ende des distalen Mandrinabschnitts hierfür in Form einer Spitze ausgebildet.

Auf einfache Weise lässt sich die medizinische Bohrvorrichtung ausbilden, wenn der Bohrvorrichtungkopplungsabschnitt spiegelsymmetrisch zu einer die Längsachse enthaltenden Spiegelebene ausgebildet ist. Insbesondere kann so ein im Querschnitt unrunder Bohrvorrichtungkopplungsabschnitt ausgebildet werden.

Ferner kann die Ausgestaltung der medizinischen Bohrvorrichtung vereinfacht werden, wenn der Bohrerkopplungsabschnitt und der proximale Mandrinabschnitt jeweils spiegelsymmetrisch zur Spiegelebene ausgebildet sind. Mit anderen Worten können der proximale Mandrinabschnitt und der Bohrerkopplungsabschnitt durch die Spiegelebene in sich selbst überführt werden. Eine solche Ausgestaltung ermöglicht insbesondere die Ausbildung eines Bohrerkopplungsabschnitts mit einem Schlitz zur Aufnahme des proximalen Mandrinabschnitts. Der Schlitz ist dann vorzugsweise ebenfalls spiegelsymmetrisch zur Spiegelebene ausgebildet.

Um Drehmomente vom Bohrerkopplungsabschnitt auf den proximalen Mandrinabschnitt oder umgekehrt sicher übertragen zu können, ist es vorteilhaft, wenn die mindestens eine Mandrinmitnahmefläche und die mindestens eine Bohrermitnahmefläche parallel zur Spiegelebene verlaufen.

Damit der Mandrin beim Bohren mit dem Bohrer mitrotiert, ist es günstig, wenn der Mandrin und der Bohrer drehfest miteinander in Eingriff stehen. Ein Rotieren des Bohrers führt dann zu einer Zwangsrotation des Mandrins.

Gemäß einer weiteren bevorzugten Ausführungsform kann vorgesehen sein, dass der Mandrin mit dem distalen Mandrinabschnitt ausgehend von einer Trennstellung, in welcher der Mandrin und der Bohrer außer Eingriff stehen, in die Bohrstellung bringbar ist durch Einführen des distalen Mandrinabschnitts in den Längskanal ausgehend von dessen proximalem Ende. Diese Ausgestaltung ermöglicht insbesondere auf einfache Weise das Koppeln des Mandrins mit dem Bohrer. Dies ist insbesondere dann vorteilhaft, wenn während eines chirurgischen Eingriffs mehrere Pedikelschrauben in der eingangs beschriebenen Weise gesetzt werden sollen. Nach dem Setzen des K-Drahts kann der kanülierte Bohrer entfernt werden. Der Mandrin kann dann wieder mit dem Bohrer in der beschriebenen Weise in Eingriff gebracht werden, um den Längskanal des Bohrers zu verschließen. So werden für einen solchen Eingriff zumindest theoretisch nur ein Bohrer mit zugeordnetem Mandrin benötigt, mithin also nur eine einzige Bohrvorrichtung.

Günstig ist es, wenn am Bohrer ein Tiefenanschlag für den Mandrin angeordnet oder ausgebildet ist zum Begrenzen einer Einführtiefe des distalen Mandrinabschnitts in den Längskanal. Auf diese Weise kann insbesondere sichergestellt werden, dass der Mandrin den Längskanal in gewünschter Weise ausfüllt. Ein Anwender muss hierfür nur den Mandrin bis zum Tiefenanschlag in den Längskanal einführen. Lässt sich der Mandrin relativ zum Bohrer in distaler Richtung nicht weiter bewegen, hat der Mandrin die gewünschte Position erreicht.

Auf einfache Weise lässt sich die Bohrvorrichtung ausbilden, wenn der Tiefenanschlag eine in proximaler Richtung weisende Anschlagfläche des Bohrers umfasst, an welcher der proximale Mandrinabschnitt in der Bohrstellung distalseitig anliegt.

Vorzugsweise erstreckt sich die Anschlagfläche quer, insbesondere senkrecht, zur Längsachse. So kann eine Tiefenanschlag auf einfache Weise realisiert werden.

Vorteilhaft ist es, wenn die Anschlagfläche an eine proximalseitige Einführöffnung des Längskanals angrenzt und diese mindestens teilweise, insbesondere vollständig, umgibt. Eine solche Ausgestaltung ermöglicht es insbesondere, den distalen Mandrinabschnitt ausschließlich korrespondierend zum Längskanal auszubilden, um den Längskanal in der Bohrstellung zu füllen. Der proximale Mandrinabschnitt kann in diesem Fall dann ausschließlich einen Teil des Bohrvorrichtungkopplungsabschnitts bilden. Ferner kann so ein Verformen, insbesondere ein Abknicken, des distalen Mandrinabschnitts relativ zum proximalen Mandrinabschnitt auf einfache Weise verhindert werden.

Vorteilhaft ist es, wenn der Bohrerkopplungsabschnitt einen sich parallel zur Längsachse erstreckenden Kopplungsschlitz umfasst und wenn der proximale Mandrinabschnitt in der Bohrstellung in den Kopplungsschlitz eingreift. Diese Ausgestaltung ermöglicht insbesondere eine einfache und definierte Relativpositionierung des proximalen Mandrinabschnitts und des Bohrerkopplungsabschnitts. Insbesondere kann so der proximale Mandrinabschnitt beidseitig durch den Bohrerkopplungsabschnitt geführt werden.

Günstig ist es, wenn der Kopplungsschlitz zwei aufeinander zu weisende Schlitzflächen aufweist und wenn die zwei Schlitzflächen die zwei Bohrermitnahmeflächen bilden. Insbesondere können die zwei Schlitzflächen parallel zueinander und parallel zur oben beschriebenen Spiegelebene ausgebildet sein. Die Spiegelebene kann in diesem Fall die Längsachse enthalten oder parallel zu dieser verlaufen.

Ferner lässt sich ein rotationsunsymmetrischer Bohrvorrichtungkopplungsabschnitt auf einfache Weise insbesondere dadurch ausbilden, dass der Bohrvorrichtungkopplungsabschnitt eine Kopplungsfläche aufweist, welche sich parallel zur Längsachse und quer, insbesondere senkrecht, zur mindestens einen Bohrermitnahmefläche erstreckt. Beispielsweise kann diese Kopplungsfläche Teil einer an einem ansonsten rotationssymmetrisch geformten Bohrvorrichtungkopplungsabschnitt ausgebildet sein.

Günstig ist es, wenn die Kopplungsfläche eben oder im Wesentlichen eben ausgebildet ist und mindestens zwei Kopplungsflächenabschnitte umfasst, wenn mindestens einer der mindestens zwei Kopplungsflächenabschnitte durch eine Seitenfläche des Bohrerkopplungsabschnitts gebildet ist und wenn mindestens ein weiterer der mindestens zwei Kopplungsflächenabschnitte durch eine Seitenfläche des proximalen Mandrinabschnitts gebildet ist. Auf diese Weise wird insbesondere ermöglicht, beim Koppeln des Bohrvorrichtungkopplungsabschnitts mit einem Handgriff für die Bohrvorrichtung oder einer Bohrmaschine Drehmomente nicht nur auf den Bohrer zu übertragen, sondern gleichzeitig auch auf den Mandrin. So kann insbesondere ein Verwinden des proximalen Mandrinabschnitts und des distalen Mandrinabschnitts relativ zueinander vermieden werden.

Auf einfache Weise lässt sich eine Kopplungsfläche ausbilden, wenn der der Bohrvorrichtungkopplungsabschnitt eine rotationssymmetrische Grundform und eine seitliche, sich parallel zur Längsachse erstreckende Abflachung aufweist.

Vorzugsweise umfasst die Abflachung die Kopplungsfläche. Eine solche Ausgestaltung ermöglicht insbesondere ein einfaches und sicheres, beispielsweise drehfestes, in Eingriff Bringen der Bohrvorrichtung mit einem Handgriff oder einer Bohrmaschine.

Gemäß einer weiteren bevorzugten Ausführungsform kann vorgesehen sein, dass der Bohrerkopplungsabschnitt und der proximale Mandrinabschnitt jeweils für sich rotationsunsymmetrisch bezogen auf die Längsachse ausgebildet sind. So lassen sich auf einfache Weise die beiden genannten Abschnitte jeweils für den anderen Abschnitt als Mitnehmer ausbilden, um Drehmomente zu übertragen.

Ferner kann es vorteilhaft sein, wenn am Bohrvorrichtungkopplungsabschnitt mindestens ein Kopplungselement angeordnet oder ausgebildet ist zum Vorgeben einer Axialposition der Bohrvorrichtung und eines Handgriffs oder einer Bohrmaschine relativ zueinander. Das mindestens eine Kopplungselement ermöglicht es insbesondere, eine Relativbewegung in axialer Richtung zwischen der Bohrvorrichtung und dem Handgriff beziehungsweise der Bohrmaschine in einer Kopplungsstellung zu verhindern.

Auf einfache Weise lassen sich ein Handgriff oder eine Bohrmaschine mit der Bohrvorrichtung koppeln, wenn das mindestens eine Kopplungselement in radialer Richtung weisend ausgebildet ist. Beispielsweise kann das mindestens eine Kopplungselement in Form einer in radialer Richtung weisender Vertiefung ausgebildet sein, beispielsweise in Form einer Ringnut oder eines bezogen auf die Längsachse eines Abschnitts einer Ringnut.

Eine Kopplung von Bohrvorrichtung und Handgriff oder Bohrmaschine lässt sich weiter vereinfachen, wenn das mindestens eine Kopplungselement in Form eines Kopplungsvorsprungs oder eines Kopplungsaufnahme ausgebildet ist. Derartige Kopplungselemente können mit korrespondierenden Kopplungselementen am Handgriff beziehungsweise an der Bohrmaschine kraft- und/oder formschlüssig in Eingriff gebracht werden.

Günstig ist es, wenn das mindestens eine Kopplungselement die Längsachse mindestens teilweise umgebend ausgebildet ist. Beispielsweise lässt sich so eine Ringnut ausbilden, die sich bezogen auf die Längsachse über einen Umfangswinkel erstreckt, welcher kleiner ist als 360°. Insbesondere dann, wenn am Bohrvorrichtungkopplungsabschnitt eine Kopplungsfläche wie oben beschrieben ausgebildet ist, wodurch der Bohrvorrichtungkopplungsabschnitt einen unrunden Querschnitt erhält, ist es nicht erforderlich, dass sich das mindestens eine Kopplungselement über einen vollen Umfang bezogen auf die Längsachse erstreckt, sondern lediglich über einen Teil des Umfangs, beispielsweise nur über einen Winkelbereich von 180° oder 270°.

Vorzugsweise ist das mindestens eine Kopplungselement durch die Abflachung unterbrochen. Mit anderen Worten ist also im Bereich der Abflachung kein Kopplungselement. Mit anderen Worten kann dies zum Beispiel erreicht werden, indem die Abflachung so dimensioniert wird, dass bei einem ursprünglich rotationssymmetrischen Bohrvorrichtungkopplungsabschnitt mit einem bezogen auf die Längsachse umlaufenden, und zwar vollständig umlaufenden Kopplungselement, das Kopplungselement im Bereich der Abflachung vollständig entfernt ist.

Überdies kann es vorteilhaft sein, wenn am proximalen Mandrinabschnitt mindestens ein Handhabungselement angeordnet ist oder ausgebildet ist. Ein solches Handhabungselement kann insbesondere dazu genutzt werden, um mit einem Finger oder einem korrespondierenden Zugelement in Eingriff gebracht zu werden, um den Mandrin relativ zum Bohrer in proximaler oder distaler Richtung zu bewegen. Insbesondere ist es denkbar, an einem Handgriff oder einem Bohrfutter einer Bohrmaschine ein zum mindestens einen Handhabungselement korrespondierendes Zugelement vorzusehen, welches gezielt mit dem mindestens einen Handhabungselement in Eingriff oder außer Eingriff gebracht werden kann, um wie beschrieben den Mandrin relativ zum Bohrer in distaler oder insbesondere in proximaler Richtung zu bewegen. Insbesondere dann, wenn der Bohrer mit dem im Längskanal aufgenommenen Mandrin im Knochen steckt, lässt sich so der Mandrin auf einfache Weise in proximaler Richtung aus dem Bohrer herausziehen.

Vorzugsweise ist das Handhabungselement in Form einer Handhabungsvertiefung oder eines Handhabungsvorsprungs ausgebildet. Beispielsweise kann das Handhabungselement durch einen Teil des mindestens einen Kopplungselements gebildet werden, welcher Teil am proximalen Mandrinabschnitt ausgebildet ist. Dies vereinfacht insbesondere den Aufbau der Bohrvorrichtung weiter.

Um auf einfache Weise in proximaler oder distaler Richtung wirkende Kräfte auf den Mandrin ausüben zu können, ist es vorteilhaft, wenn das mindestens eine Handhabungselement bezogen auf die Längsachse in radialer Richtung weisend ausgebildet ist. Beispielsweise kann das mindestens eine Handhabungselement als Teil einer bezogen auf die Längsachse umlaufenden Nut am Bohrvorrichtungkopplungsabschnitt ausgebildet sein.

Die Handhabung der Bohrvorrichtung kann weiter vereinfacht werden, wenn sie in der Bohrstellung eine quer, insbesondere senkrecht, zur Längsachse verlaufende Endfläche aufweist. Insbesondere kann diese anzeigen, ob der Mandrin in gewünschter Weise mit dem Bohrer in Eingriff steht, also insbesondere mit dem distalen Mandrinabschnitt den Längskanal des Bohrers ausfüllt.

Ferner ist es günstig, wenn der proximale Mandrinabschnitt eine proximale Mandrinendfläche definiert, wenn der Bohrerkopplungsabschnitt eine proximale Bohrerendfläche definiert und wenn die Endfläche zum Teil durch die Mandrinendfläche und zum Teil durch die Bohrerendfläche gebildet ist. Insbesondere ist dann so für einen Anwender unmittelbar erkennbar, ob der Mandrin und der Bohrer in gewünschter Weise in Eingriff stehen. Die Endfläche kann somit als eine Art optische Anzeige für einen Anwender genutzt werden.

Um eine hinreichend stabile Bohrvorrichtung ausbilden zu können, ist es vorteilhaft, wenn der Bohrer aus einem metallischen Werkstoff ausgebildet ist. Insbesondere kann er aus Edelstahl oder aus Tian ausgebildet sein.

Überdies ist es günstig, wenn der Mandrin aus einem metallischen Werkstoff oder aus einem Kunststoff ausgebildet ist. Ein Mandrin aus Kunststoff lässt sich insbesondere kostengünstig herstellen. Ein Mandrin aus einem metallischen Werkstoff kann insbesondere beim Bohren entstehende Wärme zusätzlich zum Bohrer gut ableiten.

Günstigerweise ist die Bohrvorrichtung aus mindestens einem sterilsierbaren Werkstoff ausgebildet. Insbesondere kann sie aus nur einem sterilisierbaren Werkstoff ausgebildet sein. Beispielsweise können der Mandrin und der Bohrer aus demselben Werkstoff ausgebildet sein.

Die eingangs gestellte Aufgabe wird ferner bei einem Bohrsystem der eingangs beschriebenen Art erfindungsgemäß dadurch gelöst, dass die medizinische Bohrvorrichtung in Form einer der oben beschriebenen vorteilhaften Ausführungsformen von Bohrvorrichtungen ausgebildet ist und dass das Bohrsystem einen Handgriff und/oder eine Bohrmaschine umfasst zum drehfesten Koppeln mit der medizinischen Bohrvorrichtung.

Mit dem Handgriff oder der Bohrmaschine lässt sich die Bohrvorrichtung in gewünschter Weise handhaben, insbesondere um einen Knochen bohrend zu eröffnen, beispielsweise zum Setzen eines K-Drahts als Führung für eine Pedikelschraube. Das Bohrsystem kann zudem mehrere Bohrvorrichtungen umfassen, die sich in Form und Größe voneinander unterscheiden. Beispielsweise können vom Bohrsystem unterschiedlichen Bohrerarten umfasst sein wie beispielsweise Spiralbohrer, Kronenbohrer, Aufbohrer, Zentrierbohrer oder Senkbohrer.

Günstigerweise umfasst das Bohrsystem mindestens einen K-Draht. Ein solcher K-Draht im Sinne dieser Anmeldung ist ein Bohr-Draht. Diese Begriffe werden daher synonym benutzt. Insbesondere kann es auch eine Mehrzahl von K-Drähten, beispielsweise mit unterschiedlichen Durchmessern und Längen, umfassen. Der K-Draht kann wie beschrieben nach Entfernen des Mandrins durch den Längskanal hindurch in den mit der Bohrvorrichtung eröffneten Knochen gesetzt werden. Hierfür muss er lang genug sein, um ein Abziehen des Bohrers zu erlauben. Nach Entfernen des Bohrers durch Abziehen in proximaler Richtung über den K-Draht, kann dann eine kanülierte Pedikelschraube über den K-Draht in distaler Richtung geschoben und an den Knochen herangeführt und in diesen eingeschraubt werden. Vorzugsweise ist der K-Draht länger als der Bohrer, um eine geführte Entnahme zu gewährleisten.

Auf einfache Weise lassen sich der Handgriff beziehungsweise die Bohrmaschine mit der Bohrvorrichtung koppeln, wenn der Handgriff und/oder die Bohrmaschine eine Kopplungseinrichtung umfassen zum kraft- und/oder formschlüssigen Koppeln mit dem Bohrvorrichtungkopplungsabschnitt in einer Kopplungsstellung.

Vorzugsweise ist die Kopplungseinrichtung von der Kopplungsstellung in eine Lösestellung bringbar, in welcher sie mit dem Bohrvorrichtungkopplungsabschnitt außer Eingriff steht. In der Lösestellung sich die Bohrvorrichtung dann einfach und sicher vom Handgriff beziehungsweise von der Bohrmaschine entfernen.

Ferner kann es vorteilhaft sein, wenn die Kopplungseinrichtung ein Zugelement umfasst, zum in Eingriff Bringen mit dem mindestens einen Handhabungselement, wenn die Kopplungseinrichtung die Lösestellung einnimmt. Wie bereits oben erwähnt kann so der Mandrin auf einfache und sichere Weise aus dem Bohrer entfernt werden. Hierfür müssen dann lediglich die Kopplungseinrichtung in die Lösestellung überführt und das Zugelement mit dem mindestens einem Handhabungselement in Eingriff gebracht werden. Werden der Handgriff beziehungsweise die Bohrmaschine in der beschriebenen Stellung von der Bohrvorrichtung abgezogen, bleiben sie noch mit dem Mandrin gekoppelt, stehen jedoch mit dem Bohrer außer Eingriff, sodass sie beim Zurückziehen in proximaler Richtung den Mandrin aus dem Längskanal des Bohrer herausziehen.

Die nachfolgende Beschreibung einer bevorzugten Ausführungsform der Erfindung dient im Zusammenhang mit der Zeichnung der näheren Erläuterung. Es zeigen:
- Figur 1:: eine schematische perspektivische Gesamtansicht eines Bohrsystems beim bohrenden Eröffnen eines Wirbels;
- Figur 2:: eine teilweise geschnittene Seitenansicht des Bohrsystems aus Figur 1;
- Figur 3:: eine teilweise durchbrochene Ansicht eines Ausführungsbeispiels einer Bohrvorrichtung in der Bohrstellung;
- Figur 4:: eine teilweise durchbrochene Explosionsdarstellung der Anordnung aus Figur 3;
- Figur 5:: vergrößerte Ansichten proximaler und distaler Enden der Bohrvorrichtung in der Bohrstellung gemäß Figur 3;
- Figur 6:: eine Längsschnittansicht der Anordnung aus Figur 5;
- Figur 7:: eine Ansicht analog Figur 3 eines weiteren Ausführungsbeispiels einer Bohrvorrichtung;
- Figur 8:: eine Ansicht analog Figur 4 der Bohrvorrichtung aus Figur 7;
- Figur 9:: eine vergrößerte Ansicht eines proximalen Endes der Bohrvorrichtung aus Figur 7;
- Figur 10:: eine Längsschnittansicht der Anordnung aus Figur 9;
- Figur 11:: eine schematische Ansicht beim Ansetzen einer Bohrvorrichtung an einen Knochen;
- Figur 12:: eine schematische, teilweise geschnittene Ansicht einer in einen Pedikel eingetriebenen Bohrvorrichtung;
- Figur 13:: eine schematische Ansicht ähnlich Figur 11 mit noch im Pedikel verbliebenen Bohrer nach Entfernen des Mandrins und Einsetzen eines K-Drahts durch den Längskanal des Bohrers in den Pedikel; und
- Figur 14:: eine schematische Ansicht ähnlich Figur 12 nach Entfernen des Bohrers über den K-Draht und Einbringen einer Pedikelschraube über den K-Draht in den Pedikel.

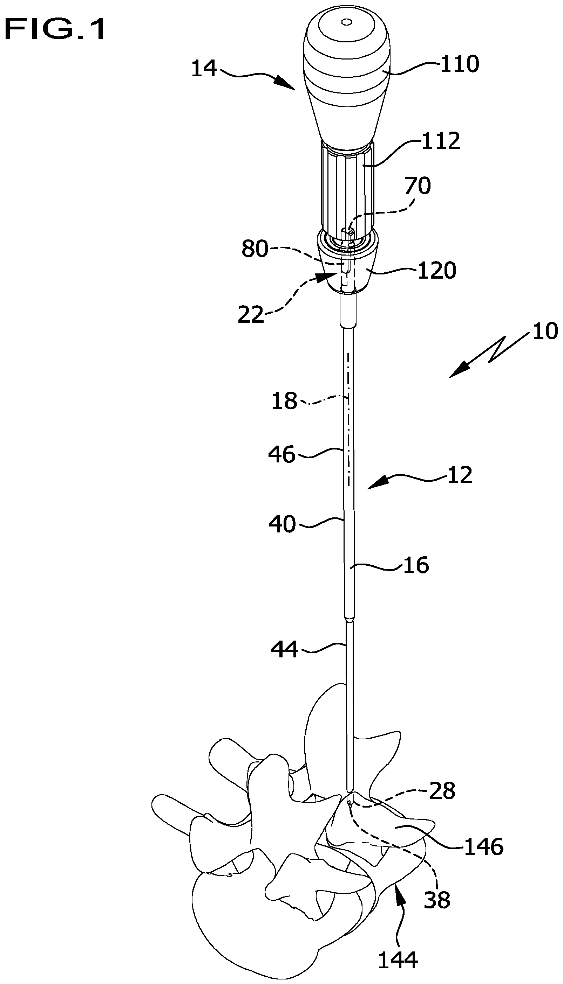

In Figur 1 ist schematisch ein Ausführungsbeispiel eines insgesamt mit dem Bezugszeichen 10 bezeichneten medizinischen Bohrsystems dargestellt. Es umfasst eine Bohrvorrichtung 12 und einen Handgriff 14 zum insbesondere drehfesten Koppeln mit der Bohrvorrichtung 12.

Die Bohrvorrichtung 12 umfasst einen kanülierten Bohrer 16 mit einem sich koaxial zu einer Längsachse 18 des Bohrers 16 erstreckenden Längskanal 20.

Die Bohrvorrichtung 12 umfasst an einem proximalen Ende einen Bohrvorrichtungkopplungsabschnitt 22 zum drehfesten Koppeln mit dem Handgriff 14 oder einer Bohrmaschine, die in den Figuren nicht dargestellt ist.

Der Bohrer 16 weist ein proximales Ende 24 und ein distales Ende 26 auf. Das distale Ende 26 ist mit mehreren Schneiden 28 versehen, die geeignet sind, insbesondere Knochen zu zerspanen.

Die Bohrvorrichtung 12 umfasst ferner einen Mandrin 30 mit einem distalen Mandrinabschnitt 32 und einem proximalen Mandrinabschnitt 34. Der distale Mandrinabschnitt 32 ist langgestreckt stabförmig ausgebildet und definiert einen kreisförmigen Querschnitt.

An einem distalen Ende 36 des distalen Mandrinabschnitts 32 ist eine Spitze 38 ausgebildet.

Der Bohrer 16 weist einen Bohrerschaft 40 auf, welcher sich ausgehend vom distalen Ende 26 in proximaler Richtung in einem Übergangsbereich 42 im Außendurchmesser einstufig erweitert. So werden ein distaler Schaftabschnitt 44 und ein proximaler Schaftabschnitt 46 definiert, zwischen denen der Übergangsbereich 42 ausgebildet ist.

Proximalseitig schließt sich an den proximalen Schaftabschnitt 46 ein Zylinderkörper 48 an. Von einer proximalen Endfläche 50 des Zylinderkörpers 48 ist ein zylindrischer Anschlagkörper 52 in proximaler Richtung abstehend ausgebildet. Der Anschlagkörper 52 weist einen etwas geringeren Außendurchmesser auf als der Zylinderkörper 48, sodass am Zylinderkörper eine in proximaler Richtung weisende Ringfläche 54 ausgebildet wird. Eine in proximaler Richtung weisende Endfläche 56 des Anschlagkörpers 52 definiert eine Anschlagfläche 58.

Von der Anschlagfläche 58 steht in proximaler Richtung weisend ein Bohrerkopplungsabschnitt 60 ab. Somit weist der Bohrer 16 im Bereich seines proximalen Endes 24 den Bohrerkopplungsabschnitt 60 auf. Der Bohrerkopplungsabschnitt 60 bildet zusammen mit dem proximalen Mandrinabschnitt 34 den Bohrvorrichtungkopplungsabschnitt 22.

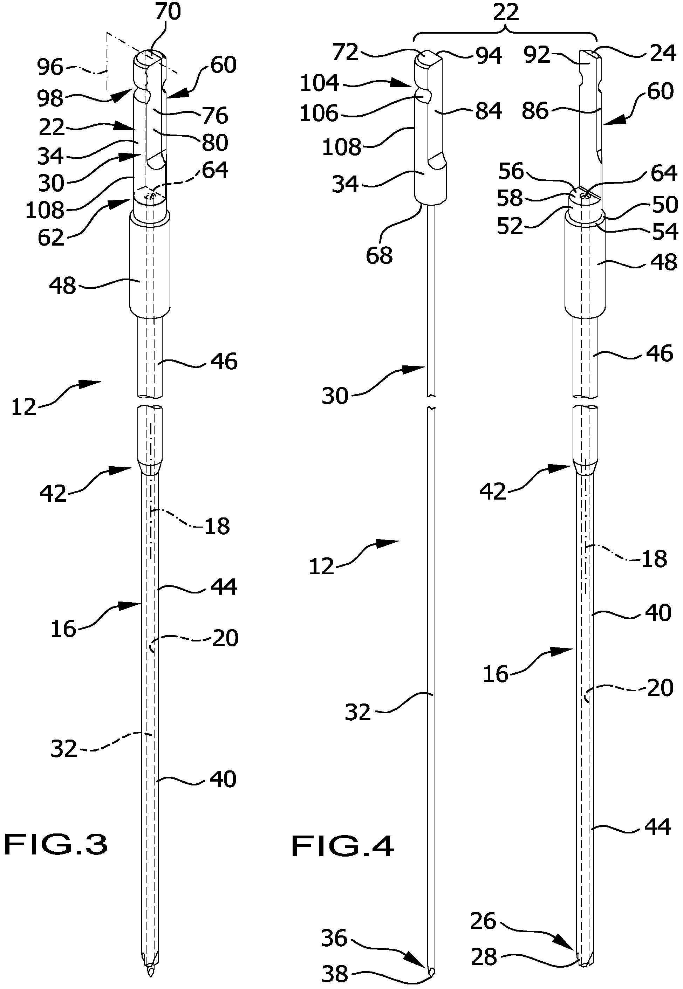

Der distale Mandrinabschnitt 32 ist ausgebildet zum Einsetzen und Verschließen des Längskanals 18 des Bohrers 16 über die gesamte Länge desselben in einer Bohrstellung, die schematisch in den Figuren 1 bis 3 sowie 5 und 6 dargestellt ist.

Ein Querschnitt des distalen Mandrinabschnitts 32 senkrecht zur Längsachse 18 korrespondiert zu einem Querschnitt des Längskanals 20, sodass der distale Mandrinabschnitt 32 den Längskanal 20 formschlüssig ausfüllt. In der Bohrstellung reicht das distale Ende 36 des distalen Mandrinabschnitts 32 bis an das distale Ende 26 des Längskanals 20 heran beziehungsweise steht etwas über das distale Ende 26 vor.

Der Längskanal 20 weist einen kreisförmigen Querschnitt auf. Bei alternativen Ausführungsbeispielen kann der Längskanal 20 auch einen ovalen oder anderweitig unrunden Querschnitt aufweisen.

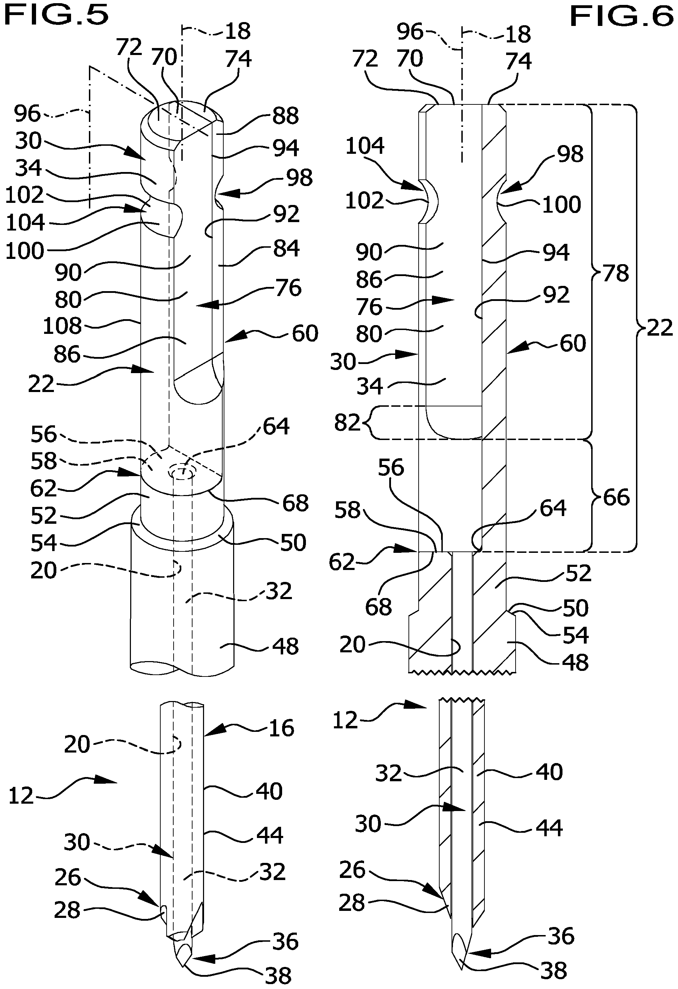

Der Anschlagkörper 52 bildet mit der Anschlagfläche 58 einen Tiefenanschlag 62 für den Mandrin 30 zum Begrenzen einen Einführtiefe des distalen Mandrinabschnitts 32 in den Längskanal 20. Der proximale Mandrinabschnitt 34 liegt in der Bohrstellung distalseitig an der in proximaler Richtung weisenden Anschlagfläche 58 des Bohrers 16 an, wie dies gut in den Figuren 5 und 6 zu erkennen ist.

Die Anschlagfläche 58 erstreckt sich quer, nämlich senkrecht, zur Längsachse 18. Sie grenzt an eine proximalseitige Einführöffnung 64 des Längskanals 20 an. Wie in Figur 5 gut zu erkennen, umgibt die Anschlagfläche 58 die Einführöffnung 64 mindestens teilweise, nämlich vollständig.

Der proximale Mandrinabschnitt 34 ergänzt sich mit dem Bohrerkopplungsabschnitt 60 im Bereich des Abschnitts 66 zu einem geraden Kreiszylinder, welcher den Anschlagkörper 52 in der Bohrstellung proximalseitig verlängert.

Der Bohrvorrichtungkopplungsabschnitt 22 erstreckt sich von einer in distaler Richtung weisenden Endfläche 68 des proximalen Mandrinabschnitts 34 bis zu einer proximalen Endfläche 70 des Bohrvorrichtungkopplungsabschnitts 22. Die Endfläche 70 erstreckt sich quer, nämlich senkrecht, zur Längsachse 18.

Die proximale Endfläche 70 ist in der Bohrstellung gebildet durch eine proximale Mandrinendfläche 72 des proximalen Mandrinabschnitts 34 und eine proximale Bohrerendfläche 74. So wird die Endfläche 68 teilweise durch die Mandrinendfläche 72 und teilweise durch die Bohrerendfläche 74 gebildet.

Der Bohrvorrichtungkopplungsabschnitt 22 weist eine zylindrische Grundform auf, die jedoch durch Ausbildung einer Abflachung 76 eine unrunde Querschnittsfläche erhält, und zwar in einem Kopplungsbereich 78, welcher sich proximalseitig an den Abschnitt 66 anschließt und bis zur Endfläche 70 reicht. Die Abflachung 76 ist seitlich ausgebildet und erstreckt sich parallel zur Längsachse 18. Die Abflachung 76 definiert so eine Kopplungsfläche 80 am Bohrvorrichtungkopplungsabschnitt 22, die sich damit ebenfalls parallel zur Längsachse 18 erstreckt.

Die Kopplungsfläche 80 ist bis auf einen kurzen Übergangsbereich 82 eben ausgebildet und umfasst zwei Kopplungsflächenabschnitt 84, 86, nämlich einen ersten Kopplungsflächenabschnitt 84, welcher durch eine Seitenfläche 88 des Bohrerkopplungsabschnitts 60 gebildet ist, und einen zweiten Kopplungsflächenabschnitt 86, welcher durch eine Seitenfläche 90 des proximalen Mandrinabschnitts 34 gebildet ist.

Durch die Ausbildung der Abflachung 76 ist der Bohrvorrichtungkopplungsabschnitt 22 bezogen auf die Längsachse 18 rotationsunsymmetrisch ausgebildet.

Der Bohrerkopplungsabschnitt 60 ist derart ausgebildet, dass er eine Bohrermitnahmefläche 92 definiert. Der proximale Mandrinabschnitt 34 definiert eine Mandrinmitnahmefläche 94. In der Bohrstellung liegen die Bohrermitnahmefläche 92 und die Mandrinmitnahmefläche 94 aneinander an, wie dies in den Figuren 5 und 6 gut zu erkennen ist.

Der Bohrvorrichtungkopplungsabschnitt 22 ist spiegelsymmetrisch zu einer die Längsachse 18 enthaltene Spiegelebene 96 ausgebildet. Die Bohrermitnahmefläche 92 und die Mandrinmitnahmefläche 94 verlaufen parallel zur Spiegelebene 96.

Durch die beschriebene Ausgestaltung des proximalen Mandrinabschnitts 34 und des Bohrerkopplungsabschnitts 60 stehen der Mandrin 30 und der Bohrer 16 in der Bohrstellung drehfest miteinander in Eingriff.

Bei diesem Ausführungsbeispiel sind sowohl der Bohrerkopplungsabschnitt 60 als auch der proximale Mandrinabschnitt 34 jeweils für sich rotationsunsymmetrisch bezogen auf die Längsachse 18 ausgebildet.

Am Bohrvorrichtungkopplungsabschnitt 22 ist ferner ein Kopplungselement 98 ausgebildet zum Vorgeben einer Axialposition der Bohrvorrichtung 12 und des Handgriffs 14 relativ zueinander. Das Kopplungselement 98 ist in radialer Richtung bezogen auf die Längsachse 18 weisend ausgebildet.

Das Kopplungselement 98 ist beim Ausführungsbeispiel der Bohrvorrichtung 12 gemäß den Figuren 1 bis 6 in Form einer Kopplungsaufnahme 100 ausgebildet. Die Kopplungsaufnahme 100 umgibt die Längsachse 18 teilweise. Wenn bei dem Bohrvorrichtungkopplungsabschnitt 22 keine Abflachung 76 vorgesehen wäre, hätte die Kopplungsaufnahme 100 die Form einer Ringnut 102. Mit anderen Worten sind diese Ringnut 102 und damit das Kopplungselement 98 durch die Abflachung 76 unterbrochen.

Das Kopplungselement 98 ist teilweise am proximalen Mandrinabschnitt 34 und teilweise am Bohrerkopplungsabschnitt 60 ausgebildet.

Am proximalen Mandrinabschnitt 34 ist zudem ein Handhabungselement 104 ausgebildet. Es weist die Form einer Handhabungsvertiefung 106 auf. Das Handhabungselement 104 ist bezogen auf die Längsachse 18 in radialer Richtung weisend ausgebildet.

Bei dem Ausführungsbeispiel der Figuren 1 bis 6 wird das Handhabungselement 104 gebildet durch den Teil des Kopplungselements 98, welcher sich entlang einer bezogen auf die Längsachse 18 äußeren Umfangsfläche 108 des proximalen Mandrinabschnitts 34 erstreckt.

Der beispielhaft dargestellte Handgriff 14 umfasst an seinem proximalen Ende einen Knauf 110, an welchen sich distalseitig ein Haltebereich 112 mit parallel zur Längsachse 18 verlaufenden Nuten 114 an. Die Nuten 114 sind gleichmäßig über einen Umfang des Haltebereichs 112 verteilt angeordnet.

Einen distalen Endbereich des Handgriffs 14 bildet eine Kopplungseinrichtung 116. Diese ist in Form einer Schnellkupplung ausgebildet mit einer auf einem Schaft 118 des Handgriffs 14 verschiebbaren Kopplungshülse 120. Die Kopplungseinrichtung 116 ermöglicht das Koppeln des Handgriffs 14 mit der Bohrvorrichtung 12 in einer Kopplungsstellung.

Die Kopplungshülse 120 ist entgegen der Wirkung einer Feder 122 in proximaler Richtung verschiebbar. Wird die zurückgeschobene Kopplungshülse 120 losgelassen, wirkt die Feder 122 derart, dass die Kopplungshülse 120 automatisch in distaler Richtung bewegt wird.

Am Schaft 118 sind mehrere in radialen Durchbrechungen 124 desselben eingesetzte Kugeln 126 gehalten. Die Kugeln 126 werden in der Kopplungsstellung, in welcher die Feder 122 die Kopplungshülse 120 in ihrer distalsten Position hält, in Richtung auf die Längsachse 18 gedrückt und stehen etwas über eine innere Wandfläche 128 einer im Schaft 118 koaxial zur Längsachse 18 ausgebildeten Kopplungsaufnahme 130 vor.

An der Kopplungshülse 120 ist an einer inneren Wandfläche 132, die in Richtung auf die Längsachse 18 hin weist, eine Vertiefung 134 in Form einer in Richtung auf die Längsachse 18 hin weisenden Ringnut ausgebildet. Diese weist eine Tiefe auf derart, dass die Kugeln 126 in die Vertiefung 134 eintauchen können, so dass sie dann die Kopplungsaufnahme 130 freigeben.

Ferner weist die Kopplungsaufnahme 130 einen sich quer zur Längsachse 18 erstreckenden inneren, rotationsunsymmetrischen Querschnitt auf, welcher an den Querschnitt des Bohrvorrichtungkopplungsabschnitts 22 angepasst ist, sodass dieser in der Kopplungsaufnahme 130 formschlüssig aufgenommen werden kann. Durch die beschriebene Ausgestaltung stehen der Bohrvorrichtungkopplungsabschnitt 22 und der Handgriff 14 drehfest miteinander in Eingriff.

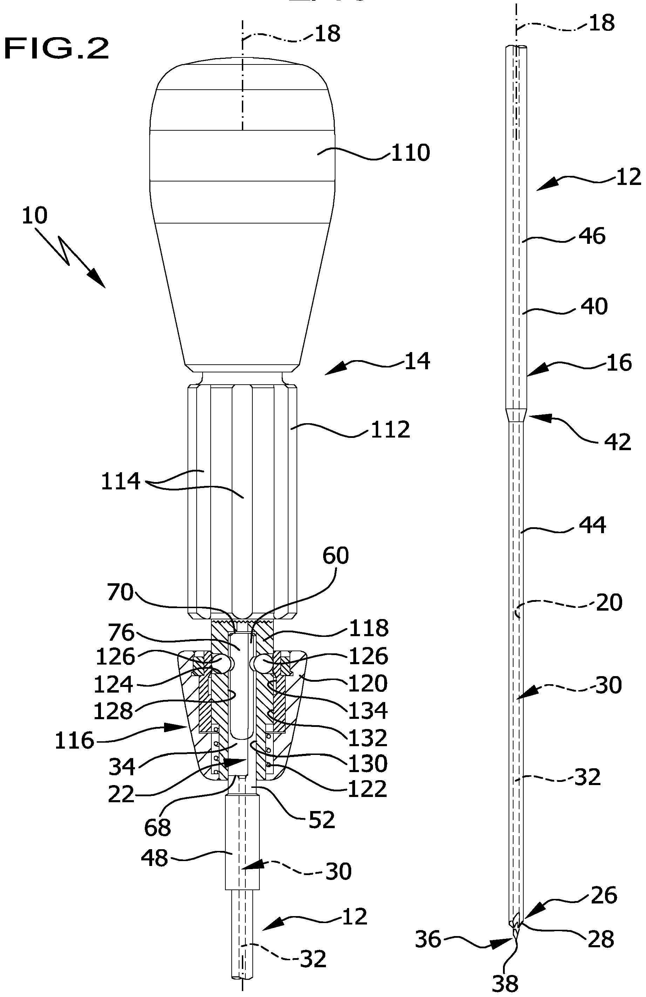

Zum Koppeln der Bohrvorrichtung 12 mit dem Handgriff 14 wird der Bohrvorrichtungkopplungsabschnitt 22 mit der proximalen Endfläche 70 voran in die distalseitig offene Kopplungsaufnahme 130 des Schafts 118 in den Handgriff 14 eingeführt. Die Kugeln 126 versperren dabei dem Bohrvorrichtungkopplungsabschnitt 22 den Weg. Wird die Kopplungshülse 120 nun in Richtung auf den Haltebereich 112 entgegen der Wirkung der Feder 122 geschoben, können die Kugel 126 wie beschrieben in die Vertiefung 134 eintauchen und der Bohrvorrichtungkopplungsabschnitt 22 weiter in proximaler Richtung verschoben werden, und zwar so weit, bis die Kopplungsaufnahme 100 den Kugeln 126 gegenüberliegt. Lässt man die Kopplungshülse 120 nun los, bewegt die Feder 122 die Kopplungshülse 120 relativ zum Haltebereich 112 in distaler Richtung, wodurch die Kugeln 126 gegen eine Bewegung in radialer Richtung von der Längsachse 18 weg gesichert werden. Figur 2 zeigt die beschriebene Kopplungsstellung, in welcher der Handgriff 14 mit seiner Kopplungseinrichtung 116 kraft- und formschlüssig mit dem Bohrvorrichtungkopplungsabschnitt 22 gekoppelt ist.

Die Kopplungseinrichtung 116 kann alternativ auch in analoger Weise an einer Bohrmaschine ausgebildet sein, sodass die Bohrvorrichtung 12 entsprechend auch mit einer Bohrmaschine gekoppelt werden kann.

Wie beschrieben ist die Kopplungseinrichtung 116 von der Kopplungsstellung in eine Lösestellung bringbar, in welcher sie mit dem Bohrvorrichtungkopplungsabschnitt 22 außer Eingriff steht. Die Lösestellung ist in den Figuren nicht dargestellt, aber oben beschrieben. In der Lösestellung ist demnach die Kopplungshülse 120 in Richtung auf den Haltebereich 112 entgegen der Wirkung der Feder 122 verschoben, sodass die Kugeln 126 in die Vertiefung 134 eintauchen und den Bohrvorrichtungkopplungsabschnitt 22 freigeben.

Optional kann die Kopplungseinrichtung 116 ein Zugelement umfassen zum in Eingriff Bringen mit dem Handhabungselement 104, und zwar dann, wenn die Kopplungseinrichtung 116 die Lösestellung einnimmt. Greift also das Zugelement nur in das Handhabungselement 104 ein, welches am proximalen Mandrinabschnitt 34 ausgebildet ist, kann die Bohrvorrichtung 12 mit Hilfe des Handgriffs 14 in seine Bestandteile zerlegt werden. Der Mandrin 30 wird beim Eingreifen des Zugelements in das Handhabungselement 104 in proximaler Richtung aus dem Längskanal 20 herausgezogen, wenn der Handgriff 14 in proximaler Richtung abgezogen wird.

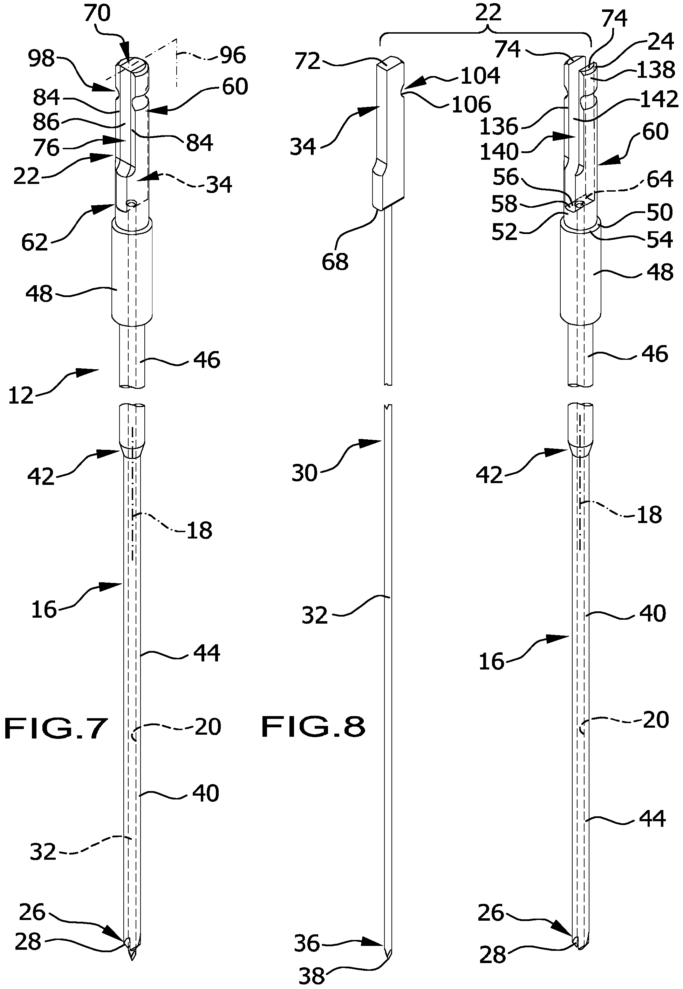

In den Figuren 7 bis 10 ist ein zweites Ausführungsbeispiel einer Bohrvorrichtung 12 schematisch dargestellt. Das Ausführungsbeispiel der Bohrvorrichtung 12 gemäß den Figuren 7 bis 10 unterscheidet sich von der Bohrvorrichtung 12 gemäß dem Ausführungsbeispiel der Figuren 1 bis 6 lediglich durch die Ausgestaltung des Bohrvorrichtungkopplungsabschnitts 22. Zur Beschreibung werden daher nachfolgend dieselben Bezugszeichen verwendet für identische oder funktional ähnliche Komponenten.

Bei diesem Ausführungsbeispiel ist der Bohrerkopplungsabschnitt 60 spiegelsymmetrisch zur Spiegelebene 96 ausgebildet und umfasst somit zwei voneinander getrennte Abschnitte 136 und 138, welche durch einen sich parallel zur Längsachse 18 erstreckenden Kopplungsschlitz 140 voneinander getrennt, nämlich beabstandet, sind. Der Kopplungsschlitz 140 weist zwei aufeinander zuweisende Schlitzflächen 142 auf, welche die Bohrermitnahmeflächen 92 bilden.

Der proximalen Mandrinabschnitt 34 ist bei diesem Ausführungsbeispiel spiegelsymmetrisch zu sich selbst bezogen auf die Spiegelebene 96 ausgebildet und greift in der Bohrstellung in den Kopplungsschlitz 140 ein derart, dass er ihn ausfüllt. Die Bohrstellung ist bei diesem Ausführungsbeispiel in den Figuren 7, 9 und 10 schematisch dargestellt.

Eine Außenkontur des Bohrvorrichtungkopplungsabschnitts 22 ist identisch mit einer Außenkontur des Bohrvorrichtungkopplungsabschnitts 22 des Ausführungsbeispiels der Figuren 1 bis 6.

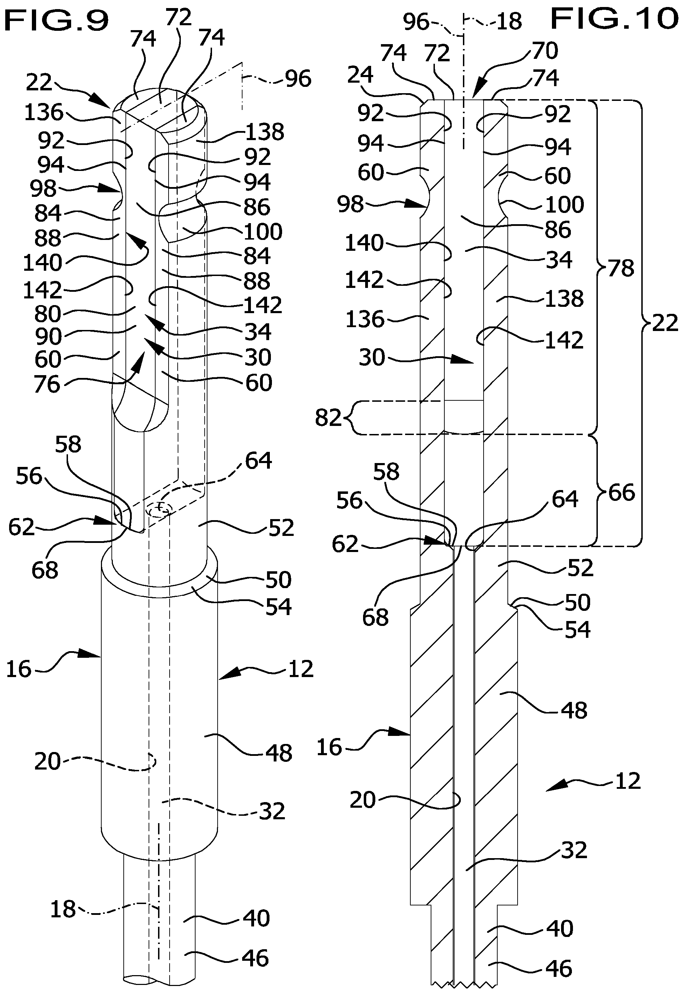

Durch die geschlitzte Ausgestaltung des Bohrerkopplungsabschnitts 60 weist dieser zwei sich parallel zur Längsachse 18 erstreckende und parallel zueinander verlaufende und aufeinander zu weisende Bohrermitnahmeflächen 92 auf. Der proximale Mandrinabschnitt 34 weist entsprechend zwei Mandrinmitnahmeflächen 94 auf. Diese weisen in entgegengesetzten Richtungen voneinander weg und verlaufen ebenfalls parallel zueinander und parallel zur Längsachse 18. Wie in Figur 10 gut zu erkennen, liegen die Bohrermitnahmeflächen 92 und die Mandrinmitnahmeflächen 94 in der Bohrstellung aneinander an.

Die Anschlagfläche 58 erstreckt sich zwischen den aufeinander zu weisenden Bohrermitnahmeflächen 92 und wird gebildet durch die Endfläche 56 des Anschlagkörpers 52.

Auch bei diesem Ausführungsbeispiel ist das Handhabungselement 104 in Form einer Handhabungsvertiefung 106 ausgebildet, und zwar als Teil des Kopplungselements 98.

Die Kopplungsfläche 80 wird beim Ausführungsbeispiel der Figuren 7 bis 10 durch zwei erste Kopplungsflächenabschnitte 84 an den Abschnitten 136 und 138 des Bohrerkopplungsabschnitts 60 sowie durch einen zweiten Kopplungsabschnitt 86 am proximalen Mandrinabschnitt 34 gebildet.

Entsprechend ist auch die proximale Endfläche 70 durch drei Teile gebildet, nämlich eine proximale Mandrinendfläche 72, welche in der Bohrstellung zwischen zwei proximalen Bohrerendflächen 74 angeordnet ist.

Die Funktionsweise der Bohrvorrichtung 12 gemäß dem Ausführungsbeispiel der Figuren 7 bis 10 entspricht vollumfänglich der Funktionsweise des Ausführungsbeispiels der Bohrvorrichtung 12 gemäß den Figuren 1 bis 6, sodass auf vorstehende Beschreibung verwiesen werden kann.

Bei den beschriebenen Ausführungsbeispielen ist der Bohrer 16 aus einem metallischen Werkstoff ausgebildet. Der Mandrin 30 ist ebenfalls aus einem metallischen Werkstoff ausgebildet.

Bei alternativen Ausführungsbeispielen ist der Mandrin 30 aus einem Kunststoff ausgebildet.

Die Bohrvorrichtung 12 ist bei allen Ausführungsbeispielen aus mindesten einem sterilisierbaren Werkstoff ausgebildet. Sie kann auch aus einem einzigen sterilisierbaren Werkstoff ausgebildet sein, beispielsweise einem sterilisierbaren metallischen Werkstoff. Sind beispielsweise der Mandrin 30 aus einem Kunststoff und der Bohrer 16 aus einem metallischen Werkstoff ausgebildet, ist die Bohrvorrichtung 12 aus zwei sterilisierbaren Werkstoffen ausgebildet.

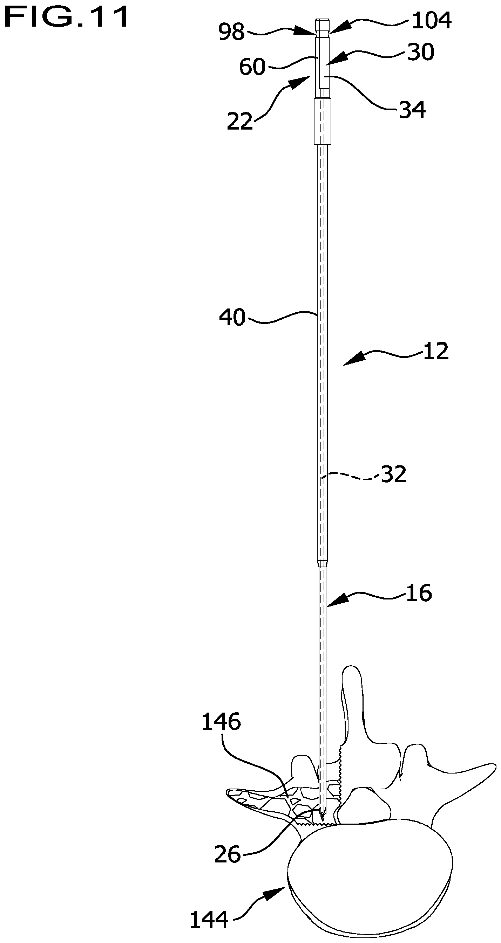

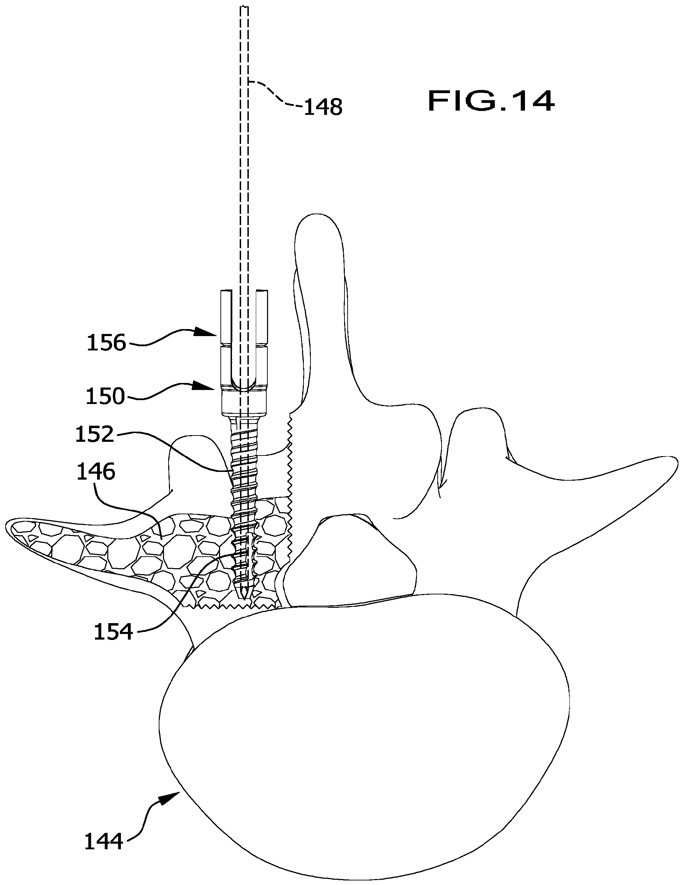

Der Einsatz des Bohrsystems 10 wird schematisch in Verbindung mit den Figuren 11 und 14 nachstehend anhand einer möglichen Anwendung in aller Kürze erläutert. Es gibt beispielsweise in der Wirbelsäulenchirurgie nicht nur Pedikelschrauben. Für die Halswirbelsäule gibt es beispielsweise auch Massa-lateralis-Schrauben. Das nachfolgend beschriebene Vorgehen stellt keine Eingrenzung des Einsatzgebiets der Bohrvorrichtung 12 für den Einsatz in Verbindung mit Pedikelschrauben 150 dar.

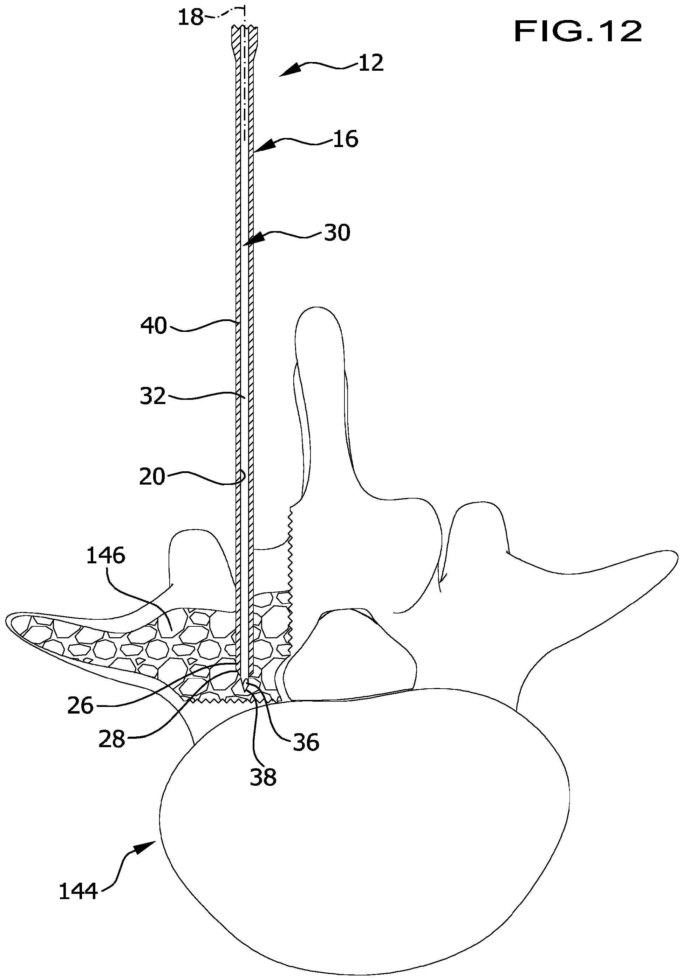

In einem ersten Schritt wird die Bohrvorrichtung 12 mit ihrem distalen Ende 26 durch einen minimal-invasiven Zugang in den Körper eines Patienten eigeführt. Die Figuren 11 und 12 zeigen diesen Vorgang schematisch, wobei aus Übersichtlichkeitsgründen weder der Handgriff 14 noch eine entsprechend geeignete Bohrmaschine dargestellt sind.

Der Bohrer 16 mit dem Mandrin 30 wird in einem Pedikel 146 des Wirbels 144 verankert. Beim Bohren verhindert der Mandrin 30, dass Knochensubstanz in den Längskanal 20 eintreten und diesen verstopfen kann.

In einem nächsten Schritt wird der Mandrin 30 aus dem Längskanal 20 des Bohrers 16 herausgezogen. Der Bohrer 16 verbleibt jedoch im Wirbel 144.

Nachdem der Mandrin 30 den Längskanal 20 freigegeben hat, kann ein vom Bohrsystem 10 umfasster K-Draht 148 von proximal her kommend durch die Einführöffnung 64 in den Längskanal 20 eingeführt und im Wirbel 144 verankert werden. Der Bohrer 16 dient dabei als Führung für den K-Draht 148.

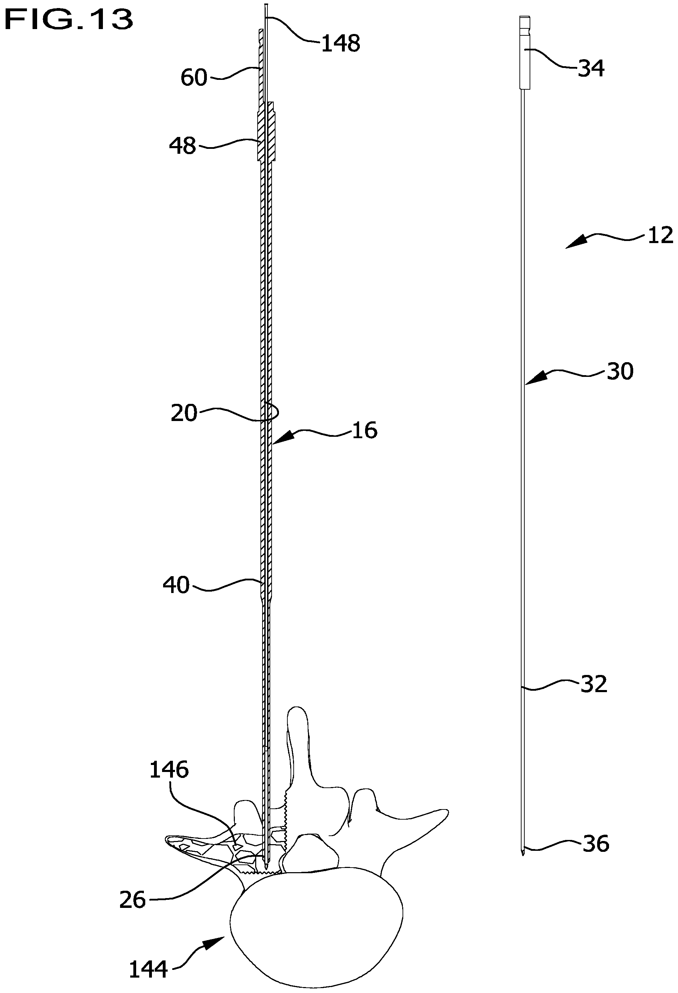

Ist der K-Draht 148 verankert, wie dies schematisch in Figur 13 dargestellt ist, kann in einem nächsten Schritt der Bohrer 16 aus dem Wirbel 144 entfernt, wobei der K-Draht 148 im Wirbel 144 verbleibt.

Der K-Draht 148 dient nun zur Führung einer kanülierten Pedikelschraube 150. Diese kann dann in üblicher Weise mit einem mit einem Außengewinde 152 versehenen Schaft 154 in den Pedikel 146 eingeschraubt werden.

Ist die Pedikelschraube 150 hinreichend im Wirbel 144 verankert, wird der K-Draht 148 entfernt. Die Pedikelschraube 150 kann nun gegebenenfalls noch weiter in den Wirbel 144 eingetrieben werden.

Ein Kopf 156 der Pedikelschraube 150 ist ausgebildet zum Aufnehmen eines in den Figuren nicht dargestellten stabförmigen Verbindungselements, welches mit einer in den Figuren nicht dargestellten Fixierschraube klemmend im Kopf 156 gesichert werden kann.

Nach dem Platzieren einer ersten Pedikelschraube 150 in der beschriebenen Weise kann der Mandrin 30 mit seinem distalen Mandrinabschnitt 32 ausgehend von einer Trennstellung, in welcher der Mandrin 30 und der Bohrer 16 vollständig außer Eingriff stehen, wieder in die Bohrstellung gebracht werden durch Einführen des distalen Mandrinabschnitts 32 in den Längskanal 20 ausgehend von dessen proximalem Ende. So ist es möglich, mit nur einer einzigen Bohrvorrichtung 12 während eines chirurgischen Eingriffs mehrere Pedikelschrauben 150 in der oben beschriebenen Wiese in Wirbeln 144 zu verankern.

### Bezugszeichenliste

- 10: Bohrsystem
- 12: Bohrvorrichtung
- 14: Handgriff
- 16: Bohrer
- 18: Längsachse
- 20: Längskanal
- 22: Bohrvorrichtungkopplungsabschnitt
- 24: proximales Ende
- 26: distales Ende
- 28: Schneide
- 30: Mandrin
- 32: distaler Mandrinabschnitt
- 34: proximaler Mandrinabschnitt
- 36: distales Ende
- 38: Spitze
- 40: Bohrerschaft
- 42: Übergangsbereich
- 44: distaler Schaftabschnitt
- 45: proximaler Schaftabschnitt
- 48: Zylinderkörper
- 50: Endfläche
- 52: Anschlagkörper
- 54: Ringfläche
- 56: Endfläche
- 58: Anschlagfläche
- 60: Bohrerkopplungsabschnitt
- 62: Tiefenanschlag
- 64: Einführöffnung
- 66: Abschnitt
- 68: Endfläche
- 70: proximale Endfläche
- 72: proximale Mandrinendfläche
- 74: proximale Bohrerendfläche
- 76: Abflachung
- 78: Kopplungsbereich
- 80: Kopplungsfläche
- 82: Übergangsbereich
- 84: erster Kopplungsflächenabschnitt
- 86: zweiter Kopplungsflächenabschnitt
- 88: Seitenfläche
- 90: Seitenfläche
- 92: Bohrermitnahmefläche
- 94: Mandrinmitnahmefläche
- 96: Spiegelebene
- 98: Kopplungselement
- 100: Kopplungsaufnahme
- 102: Ringnut
- 104: Handhabungselement
- 106: Handhabungsvertiefung
- 108: Umfangsfläche
- 110: Knauf
- 112: Haltebereich
- 114: Nut
- 116: Kopplungseinrichtung
- 118: Schaft
- 120: Kopplungshülse
- 122: Feder
- 124: Durchbrechung
- 126: Kugel
- 128: Wandfläche
- 130: Kopplungsaufnahme
- 132: Wandfläche
- 134: Vertiefung
- 136: Abschnitt
- 138: Abschnitt
- 140: Kopplungsschlitz
- 142: Schlitzfläche
- 144: Wirbel
- 146: Pedikel
- 148: K-Draht
- 150: Pedikelschraube
- 152: Außengewinde
- 154: Schaft
- 156: Kopf

## Patentansprüche

1. Medizinische Bohrvorrichtung (12) umfassend einen kanülierten Bohrer (16) mit einem sich koaxial zur Längsachse (18) des Bohrers (16) erstreckenden Längskanal (20), wobei die Bohrvorrichtung (12) an einem proximalen Ende einen Bohrvorrichtungkopplungsabschnitt (22) umfasst zum drehfesten Koppeln mit einem Handgriff (14) oder einer Bohrmaschine, wobei der Bohrer (16) ein proximales und ein distales Ende (24, 26) aufweist, wobei die Bohrvorrichtung (12) einen Mandrin (30) umfasst mit einem distalen Mandrinabschnitt (32) und einem proximalen Mandrinabschnitt (34), wobei der distale Mandrinabschnitt (32) ausgebildet ist zum Einsetzen und Verschließen des Längskanals (20) des Bohrers (16) über die gesamte oder im Wesentlichen die gesamte Länge desselben in einer Bohrstellung und wobei der Bohrer (16) im Bereich seines proximalen Endes (24) einen Bohrerkopplungsabschnitt (60) umfasst, **dadurch gekennzeichnet, dass** der Bohrerkopplungsabschnitt (60) zusammen mit dem proximalen Mandrinabschnitt (34) den Bohrvorrichtungkopplungsabschnitt (22) ausbildet.

2. Medizinische Bohrvorrichtung nach Anspruch 1, **dadurch gekennzeichnet, dass** der Bohrvorrichtungkopplungsabschnitt (22) bezogen auf die Längsachse (18) rotationsunsymmetrisch ausgebildet ist.

3. Medizinische Bohrvorrichtung nach Anspruch 1 oder 2, **dadurch gekennzeichnet, dass** der Bohrerkopplungsabschnitt (60) mindestens eine Bohrermitnahmefläche (92) aufweist, dass der proximale Mandrinabschnitt (34) mindestens eine Mandrinmitnahmefläche (94) aufweist und dass die mindestens eine Bohrermitnahmefläche (92) und die mindestens eine Mandrinmitnahmefläche (94) in der Bohrstellung aneinander anliegen oder im Wesentlichen aneinander anliegen,
wobei insbesondere der Bohrerkopplungsabschnitt (60) zwei Bohrermitnahmeflächen (92) aufweist und dass der proximale Mandrinabschnitt (34) zwei Mandrinmitnahmeflächen (94) aufweist.

4. Medizinische Bohrvorrichtung nach einem der voranstehenden Ansprüche, **dadurch gekennzeichnet, dass** der distale Mandrinabschnitt (32) einen Querschnitt aufweist, welcher zum Querschnitt des Längskanals (20) korrespondiert.

5. Medizinische Bohrvorrichtung nach einem der voranstehenden Ansprüche, **dadurch gekennzeichnet, dass** der Bohrvorrichtungkopplungsabschnitt (22) spiegelsymmetrisch zu einer die Längsachse (18) enthaltenden Spiegelebene (96) ausgebildet ist,
wobei insbesondere
a) der Bohrerkopplungsabschnitt (60) und der proximale Mandrinabschnitt (34) jeweils spiegelsymmetrisch zur Spiegelebene (96) ausgebildet sind
und/oder
b) die mindestens eine Mandrinmitnahmefläche (94) und die mindestens eine Bohrermitnahmefläche (92) parallel zur Spiegelebene (96) verlaufen.

6. Medizinische Bohrvorrichtung nach einem der voranstehenden Ansprüche, **dadurch gekennzeichnet, dass** der Mandrin (30) und der Bohrer (16) in der Bohrstellung drehfest miteinander in Eingriff stehen.

7. Medizinische Bohrvorrichtung nach einem der voranstehenden Ansprüche, **dadurch gekennzeichnet, dass** am Bohrer (16) ein Tiefenanschlag (62) für den Mandrin (30) angeordnet oder ausgebildet ist zum Begrenzen einer Einführtiefe des distalen Mandrinabschnitts (32) in den Längskanal (20),
wobei insbesondere der Tiefenanschlag (62) eine in proximaler Richtung weisende Anschlagfläche (58) des Bohrers (16) umfasst, an welcher der proximale Mandrinabschnitt (34) in der Bohrstellung distalseitig anliegt.

8. Medizinische Bohrvorrichtung nach einem der voranstehenden Ansprüche, **dadurch gekennzeichnet, dass** der Bohrerkopplungsabschnitt (60) einen sich parallel zur Längsachse (18) erstreckenden Kopplungsschlitz (140) umfasst und dass der proximale Mandrinabschnitt (34) in der Bohrstellung in den Kopplungsschlitz (140) eingreift,
wobei insbesondere der Kopplungsschlitz (140) zwei aufeinander zu weisende Schlitzflächen (142) aufweist und wobei die zwei Schlitzflächen (142) die zwei Bohrermitnahmeflächen (92) bilden.

9. Medizinische Bohrvorrichtung nach einem der voranstehenden Ansprüche, **dadurch gekennzeichnet, dass** der Bohrvorrichtungkopplungsabschnitt (22) eine Kopplungsfläche (80) aufweist, welche sich parallel zur Längsachse (18) und quer, insbesondere senkrecht, zur mindestens einen Bohrermitnahmefläche (92) erstreckt,
wobei insbesondere die Kopplungsfläche (80) eben oder im Wesentlichen eben ausgebildet ist und mindestens zwei Kopplungsflächenabschnitte (84, 86) umfasst, wobei mindestens einer der mindestens zwei Kopplungsflächenabschnitte (84, 86) durch eine Seitenfläche (88) des Bohrerkopplungsabschnitts (60) gebildet ist und wobei mindestens ein weiterer der mindestens zwei Kopplungsflächenabschnitte (84, 86) durch eine Seitenfläche (88) des proximalen Mandrinabschnitts (34) gebildet ist.

10. Medizinische Bohrvorrichtung nach einem der voranstehenden Ansprüche, **dadurch gekennzeichnet, dass** der Bohrvorrichtungkopplungsabschnitt (22) eine rotationssymmetrische Grundform und eine seitliche, sich parallel zur Längsachse (18) erstreckende Abflachung (76) aufweist, wobei insbesondere die Abflachung (76) die Kopplungsfläche (80) umfasst.

11. Medizinische Bohrvorrichtung nach einem der voranstehenden Ansprüche, **dadurch gekennzeichnet, dass** der Bohrerkopplungsabschnitt (60) und der proximale Mandrinabschnitt (34) jeweils für sich rotationsunsymmetrisch bezogen auf die Längsachse (18) ausgebildet sind.

12. Medizinische Bohrvorrichtung nach einem der voranstehenden Ansprüche, **dadurch gekennzeichnet, dass** am Bohrvorrichtungkopplungsabschnitt (22) mindestens ein Kopplungselement (98) angeordnet oder ausgebildet ist zum Vorgeben einer Axialposition der Bohrvorrichtung (12) und eines Handgriffs (14) oder einer Bohrmaschine relativ zueinander,
wobei insbesondere das mindestens eine Kopplungselement (98)
a) in radialer Richtung weisend ausgebildet ist
und/oder
b) in Form eines Kopplungsvorsprungs oder einer Kopplungsaufnahme (100) ausgebildet ist
und/oder
c) die Längsachse (18) mindestens teilweise umgebend ausgebildet ist
und/oder
d) durch die Abflachung (76) unterbrochen ist.

13. Medizinische Bohrvorrichtung nach einem der voranstehenden Ansprüche, **dadurch gekennzeichnet, dass** am proximalen Mandrinabschnitt (34) mindestens ein Handhabungselement (104) angeordnet oder ausgebildet ist.

14. Medizinisches Bohrsystem (10) umfassend mindestens eine medizinische Bohrvorrichtung (12) nach einem der voranstehenden Ansprüche und einen Handgriff und/oder eine Bohrmaschine zum drehfesten Koppeln mit der medizinischen Bohrvorrichtung (12).

15. Medizinisches Bohrsystem nach Anspruch 14, **dadurch gekennzeichnet, dass**
a) es mindestens einen K-Draht (148)
und/oder
b) der Handgriff (14) und/oder die Bohrmaschine eine Kopplungseinrichtung (116) umfassen zum kraft- und/oder formschlüssigen Koppeln mit dem Bohrvorrichtungkopplungsabschnitt (22) in einer Kopplungsstellung,
wobei insbesondere die Kopplungseinrichtung (116) von der Kopplungsstellung in eine Lösestellung bringbar ist, in welcher sie mit dem Bohrvorrichtungkopplungsabschnitt (22) außer Eingriff steht, wobei weiter insbesondere die Kopplungseinrichtung (116) ein Zugelement umfasst zum in Eingriff Bringen mit dem mindestens einen Handhabungselement (104), wenn die Kopplungseinrichtung (116) die Lösestellung einnimmt.

## Claims

1. Medical drilling apparatus (12) comprising a cannulated drill (16) having a longitudinal channel (20) extending coaxially with the longitudinal axis (18) of the drill (16), wherein the drilling apparatus (12) comprises at a proximal end a drilling apparatus coupling portion (22) for coupling to a handle (14) or a drilling machine in a rotationally fixed manner, wherein the drill (16) has a proximal and a distal end (24, 26), wherein the drilling apparatus (12) comprises a mandrin (30) with a distal mandrin portion (32) and a proximal mandrin portion (34), wherein the distal mandrin portion (32) is configured for inserting into and closing the longitudinal channel (20) of the drill (16) over the entire or substantially the entire length thereof in a drilling position, and wherein the drill (16) comprises in the region of its proximal end (24) a drill coupling portion (60), **characterized in that** the drill coupling portion (60) together with the proximal mandrin portion (34) forms the drilling apparatus coupling portion (22).

2. Medical drilling apparatus in accordance with Claim 1, **characterized in that** the drilling apparatus coupling portion (22) is of rotationally asymmetrical configuration with respect to the longitudinal axis (18).

3. Medical drilling apparatus in accordance with Claim 1 or 2, **characterized in that** the drill coupling portion (60) has at least one drill entraining face (92), **in that** the proximal mandrin portion (34) has at least one mandrin entraining face (94), and **in that** the at least one drill entraining face (92) and the at least one mandrin entraining face (94) abut against one another or substantially abut against one another in the drilling position, wherein, in particular, the drill coupling portion (60) has two drill entraining faces (92), and wherein the proximal mandrin portion (34) has two mandrin entraining faces (94).

4. Medical drilling apparatus in accordance with any one of the preceding Claims, **characterized in that** the distal mandrin portion (32) has a cross-section that corresponds to the cross-section of the longitudinal channel (20).

5. Medical drilling apparatus in accordance with any one of the preceding Claims, **characterized in that** the drilling apparatus coupling portion (22) is of mirror-symmetrical configuration relative to a mirror plane (96) containing the longitudinal axis (18),
wherein, in particular,
a) the drill coupling portion (60) and the proximal mandrin portion (34) are each of mirror-symmetrical configuration relative to the mirror plane (96),
and/or
b) the at least one mandrin entraining face (94) and the at least one drill entraining face (92) extend in parallel to the mirror plane (96).

6. Medical drilling apparatus in accordance with any one of the preceding Claims, **characterized in that** the mandrin (30) and the drill (16) are in engagement with one another in a rotationally fixed manner in the drilling position.

7. Medical drilling apparatus in accordance with any one of the preceding Claims, **characterized in that** a depth stop (62) for the mandrin (30) is arranged or formed on the drill (16) for delimiting an insertion depth of the distal mandrin portion (32) into the longitudinal channel (20),
wherein, in particular, the depth stop (62) comprises a stop face (58) of the drill (16) pointing in the proximal direction, against which stop face the proximal mandrin portion (34) abuts on the distal side in the drilling position.

8. Medical drilling apparatus in accordance with any one of the preceding Claims, **characterized in that** the drill coupling portion (60) comprises a coupling slot (140) extending in parallel to the longitudinal axis (18), and **in that** the proximal mandrin portion (34) engages into the coupling slot (140) in the drilling position,
wherein, in particular, the coupling slot (140) has two mutually facing slot faces (142), and wherein the two slot faces (142) form the two drill entraining faces (92).

9. Medical drilling apparatus in accordance with any one of the preceding Claims, **characterized in that** the drilling apparatus coupling portion (22) has a coupling face (80), which extends in parallel to the longitudinal axis (18) and transversely, in particular perpendicularly, to the at least one drill entraining face (92),
wherein, in particular, the coupling face (80) is of planar or substantially planar configuration and comprises at least two coupling face portions (84, 86), wherein at least one of the at least two coupling face portions (84, 86) is formed by a side face (88) of the drill coupling portion (60), and wherein at least one further one of the at least two coupling face portions (84, 86) is formed by a side face (88) of the proximal mandrin portion (34).

10. Medical drilling apparatus in accordance with any one of the preceding Claims, **characterized in that** the drilling apparatus coupling portion (22) has a rotationally symmetrical basic shape and a lateral flattened portion (76) extending in parallel to the longitudinal axis (18),
wherein, in particular, the flattened portion (76) comprises the coupling face (80).

11. Medical drilling apparatus in accordance with any one of the preceding Claims, **characterized in that** the drill coupling portion (60) and the proximal mandrin portion (34) are each individually of rotationally asymmetrical configuration with respect to the longitudinal axis (18).

12. Medical drilling apparatus in accordance with any one of the preceding Claims, **characterized in that** at least one coupling element (98) is arranged or formed on the drilling apparatus coupling portion (22) for predetermining an axial position of the drilling apparatus (12) and a handle (14) or a drilling machine relative to one another,
wherein, in particular, the at least one coupling element (98)
a) is configured pointing in the radial direction
and/or
b) is configured in the form of a coupling projection or a coupling receptacle (100)
and/or
c) is configured at least partially surrounding the longitudinal axis (18)
and/or
d) is interrupted by the flattened portion (76).

13. Medical drilling apparatus in accordance with any one of the preceding Claims, **characterized in that** at least one handling element (104) is arranged or formed on the proximal mandrin portion (34).

14. Medical drilling system (10) comprising at least one medical drilling apparatus (12) in accordance with any one of the preceding Claims and comprising a handle and/or a drilling machine for coupling to the medical drilling apparatus (12) in a rotationally fixed manner (12).

15. Medical drilling system in accordance with Claim 14, **characterized in that**
a) it comprises at least one K-wire (148)
and/or
b) the handle (14) and/or the drilling machine comprise a coupling device (116) for coupling to the drilling apparatus coupling portion (22) in a force-locking and/or positive-locking manner in a coupling position,
wherein, in particular, the coupling device (116) is bringable from the coupling position into a release position in which it is out of engagement with the drilling apparatus coupling portion (22),
wherein, further in particular, the coupling device (116) comprises a pulling element for being brought into engagement with the at least one handling element (104) when the coupling device (116) adopts the release position.

## Revendications

1. Dispositif de perçage médical (12) comprenant un foret canulé (16) doté d'un canal longitudinal (20) s'étendant coaxialement à l'axe longitudinal (18) du foret (16), le dispositif de perçage (12) comprenant, à une extrémité proximale, une section de couplage du dispositif de perçage (22) pour un couplage non rotatif avec une poignée (14) ou une machine de perçage, le foret (16) ayant une extrémité proximale et une extrémité distale (24, 26), le dispositif de perçage (12) comprenant un mandrin (30) ayant une section de mandrin distale (32) et une section de mandrin proximale (34), dans lequel la section de mandrin distale (32) est adaptée pour permettre l'insertion et la fermeture du canal longitudinal (20) du foret (16) sur toute ou sensiblement toute la longueur de celui-ci dans une position de perçage, et dans lequel le foret (16) comprend une section de couplage de foret (60) dans la zone de son extrémité proximale (24), **caractérisé en ce que** la section de couplage de foret (60) forme, avec la section de mandrin proximale (34), la section de couplage du dispositif de perçage (22).

2. Dispositif de perçage médical selon la revendication 1, **caractérisé en ce que** la section de couplage du dispositif de perçage (22) est conçue avec une asymétrie de révolution par rapport à l'axe longitudinal (18).

3. Dispositif de perçage médical selon la revendication 1 ou 2, **caractérisé en ce que** la section de couplage de foret (60) présente au moins une surface d'entraînement de foret (92), **en ce que** la section de mandrin proximal (34) présente au moins une surface d'entraînement de mandrin (94) et **en ce que** l'au moins une surface d'entraînement de foret (92) et l'au moins une surface d'entraînement de mandrin (94) sont en contact ou sensiblement en contact l'une avec l'autre dans la position de perçage,
dans lequel, en particulier, la section de couplage de foret (60) présente deux surfaces d'entraînement de foret (92) et **en ce que** la section de mandrin proximale (34) présente deux surfaces d'entraînement de mandrin (94).

4. Dispositif de perçage médical selon l'une des revendications précédentes, **caractérisé en ce que** la section distale du mandrin (32) présente une section transversale qui correspond à la section transversale du canal longitudinal (20).

5. Dispositif de perçage médical selon l'une quelconque des revendications précédentes, **caractérisé en ce que** la section de couplage du dispositif de perçage (22) est réalisée avec une symétrie par réflexion par rapport à un plan miroir (96) contenant l'axe longitudinal (18),
dans lequel, en particulier
a) la section de couplage de foret (60) et la section de mandrin proximal (34) sont chacune symétriques par rapport au plan miroir (96)
et/ou
b) l'au moins une surface d'entraînement de mandrin (94) et l'au moins une surface d'entraînement de foret (92) s'étendent parallèlement au plan miroir (96).

6. Dispositif de perçage médical selon l'une des revendications précédentes, **caractérisé en ce que** le mandrin (30) et le foret (16) sont en prise solidaire de manière non rotative l'un avec l'autre dans la position de perçage.

7. Dispositif de perçage médical selon l'une des revendications précédentes, **caractérisé en ce qu'**une butée de profondeur (62) pour le mandrin (30) est agencée ou formée sur le foret (16) pour limiter une profondeur d'introduction de la section du mandrin distale (32) dans le canal longitudinal (20),
dans lequel, en particulier, la butée de profondeur (62) comprenant une surface de butée (58) du foret (16) orientée dans la direction proximale, contre laquelle la section de mandrin proximale (34) s'appuie du côté distal dans la position de perçage.

8. Dispositif de perçage médical selon l'une des revendications précédentes, **caractérisé en ce que** la section de couplage de foret (60) comprend une fente de couplage (140) s'étendant parallèlement à l'axe longitudinal (18) et **en ce que** la section de mandrin proximale (34) s'engage dans la fente de couplage (140) dans la position de perçage,
dans lequel, en particulier, la fente de couplage (140) présentant deux surfaces de fentes (142) orientées l'une vers l'autre et dans lequel les deux surfaces de fentes (142) formant les deux surfaces d'entraînement du foret (92).

9. Dispositif de perçage médical selon l'une des revendications précédentes, **caractérisé en ce que** la section de couplage du dispositif de perçage (22) présente une surface de couplage (80) qui s'étend parallèlement à l'axe longitudinal (18) et transversalement, en particulier perpendiculairement, à au moins une surface d'entraînement du foret (92),
dans lequel, en particulier, la surface de couplage (80) étant plane ou essentiellement plane et comprenant au moins deux sections de surface de couplage (84, 86), dans lequel au moins une des au moins deux sections de surface de couplage (84, 86) étant formée par une surface latérale (88) de la section de couplage du foret (60) et dans lequel au moins une autre des au moins deux sections de surface de couplage (84, 86) étant formée par une surface latérale (88) de la section de mandrin proximale (34).

10. Dispositif de perçage médical selon l'une des revendications précédentes, **caractérisé en ce que** la section de couplage du dispositif de perçage (22) présente une forme de base à symétrie de révolution et une partie plate latérale (76) s'étendant parallèlement à l'axe longitudinal (18), la partie plate (76) comprenant notamment la surface de couplage (80).

11. Dispositif de perçage médical selon l'une des revendications précédentes, **caractérisé en ce que** la section de couplage de foret (60) et la section de mandrin proximale (34) sont réalisées chacune en soi avec une asymétrie de révolution par rapport à l'axe longitudinal (18).

12. Dispositif de perçage médical selon l'une des revendications précédentes, **caractérisé en ce qu'**au moins un élément de couplage (98) est disposé ou agencé sur la section de couplage du dispositif de perçage (22) pour prédéfinir une position axiale du dispositif de perçage (12) et d'une poignée (14) ou d'une machine de perçage l'un par rapport à l'autre, dans lequel, en particulier, l'au moins un élément de couplage (98)
a) est réalisé en étant orienté dans la direction radiale et/ou
b) est réalisé sous la forme d'une saillie de couplage ou d'un logement de couplage (100)
et/ou
c) est réalisé de manière à entourer au moins partiellement l'axe longitudinal (18)
et/ou
d) est interrompu par la partie plate (76).

13. Dispositif de perçage médical selon l'une des revendications précédentes, **caractérisé en ce qu'**au moins un élément de manipulation (104) est agencé ou formé sur la section proximale du mandrin (34).

14. Système de perçage médical (10) comprenant au moins un dispositif de perçage médical (12) selon l'une des revendications précédentes et une poignée et/ou une machine de perçage pour le couplage non rotatif avec le dispositif de perçage médical (12).

15. Système de perçage médical selon la revendication 14, **caractérisé en ce que**
a) il comprend au moins un câble K (148)
et/ou
b) la poignée (14) et/ou la machine de perçage comprennent un dispositif de couplage (116) pour le couplage par force et/ou par emboîtement avec la section de couplage du dispositif de perçage (22) dans une position de couplage,
dans lequel, en particulier, le dispositif de couplage (116) peut être amené de la position de couplage à une position de déblocage dans laquelle il est dégagé de la section de couplage du dispositif de perçage (22),
dans lequel, en outre, en particulier, le dispositif de couplage (116) comprend un élément de traction destiné à venir en prise avec l'au moins un élément de manipulation (104) lorsque le dispositif de couplage (116) prend la position de déblocage.
